# EUROPEAN PATENT APPLICATION

(11) **EP 2 380 580 A2**
(43) Date of publication of application: **26.10.2011**
(21) Application number: 10731413.0
(22) Date of filing: 18.01.2010
(51) Int. Cl.: A61K 36/074, A61K 36/07, A61P 37/00, A61K 36/258

(54) **COMPOSITION CONTAINING EXTRACTS OF FUSCOPORIA OBLIQUA, GANODERMA LUCIDUM AND PHELLINUS LINTEUS FOR PROMOTING THE PROLIFERATION OF HEMATOPOIETIC STEM CELLS**

(30) Priority: 19.01.2009 KR 20090004151
(71) Applicant: RNL Bio Co., Ltd., Gwanak-gu Seoul 151-050 (KR)
(72) Inventor: BAE, Hyung Suk, Gunpo-si Gyeonggi-do 435-040 (KR); KANG, Sung Keun, Seoul 151-050 (KR); SHIN, Il Seob, Seoul 151-762 (KR); WOO, Sang Kyu, Anyang-si Gyeonggi-do 431-080 (KR); KIM, Yun Jung, Seoul 137-130 (KR); KIM, Mi Ae, Suwon-si Gyeonggi-do 442-150 (KR); RA, Jeong Chan, Suwon-si Gyeonggi-do 440-300 (KR)
(74) Representative: Hiebl, Inge Elisabeth
(86) International application number: PCT/KR2010/000310
(87) International publication number: WO 2010/082798

(57) **Abstract**

The present invention relates to a composition for promoting the proliferation of hematopoietic stem cells, which contains mushroom extracts, and more particularly to a composition and functional food for promoting the proliferation of hematopoietic stem cells, which contain, as active ingredients, an *Inonotus Obliquus* extract, a *Ganoderma Luciderm* extract, a *Phellinus Linteus* extract, and plant extracts promoting the immune activity-enhancing effects of these mushroom extracts. The composition and functional food of the invention assists in enhancing immune activity by significantly promoting the proliferation of hematopoietic stem cells, and shows positive effects on the increase in anticancer activity, anti-inflammatory activity and cell activity, the regeneration of damaged tissue and the prevention of cardiovascular diseases, through the proliferation of hematopoietic stem cells and the enhancement of immune activity.

## Description

### TECHNICAL FIELD

The present invention relates to a composition for promoting the proliferation of hematopoietic stem cells, which contains mushroom extracts, and more particularly to a composition and functional food for promoting the proliferation of hematopoietic stem cells, which contain, as active ingredients, an *Inonotus Obliquus* extract, a *Ganoderma Luciderm* extract, a *Phellinus Linteus* extract, and plant extracts promoting the immune activity-enhancing effects of these mushroom extracts.

### BACKGROUND ART

Immunity is classified into innate immunity which is inborn, and acquired immunity which is developed during the growth of an individual. Innate immunity which is also called "native immunity" responds nonspecifically to antigens and has no specific memory. The innate immune systems include the skin functioning as a barrier against antigens, mucous tissue, highly acidic gastric acid, complements in blood, etc. Innate immune cells include macrophages, polymorphonuclear leukocytes, killer cells capable of killing infected cells, and the like. Indeed, the innate immunity system defends the body against most infections. Meanwhile, the acquired immunity system can recognize and respond specifically to an antigen that previously invaded the body, and effectively remove the antigen. Thus, acquired immunity functions to enhance innate immunity. For convenience, the acquired immunity is subdivided into humoral immunity and cell-mediated immunity.

In humoral immunity, B lymphocytes recognize antigens and then differentiate to secrete antibodies that function to remove mainly infectious bacteria. The antibodies are present in humors and consist of glycoproteins, called "immunoglobulins" (hereinafter, referred to as Igs), including IgG, IgM, IgM, IgA, IgD and IgE, which perform unique functions that can also overlap partially. IgA antibody is characterized in that it is delivered to the fetus through the placenta. This immunity is referred to as "maternal immunity" and provides protection from infection for a few months after birth. Meanwhile, in cell-mediated immunity, T-lymphocytes derived from the thymus recognize antigens and secrete lymphokines or kill infected cells directly. The secreted lymphokines may assist in phagocytosis by activating macrophages. This cell-mediated immunity mainly functions to remove cells infected by viruses or bacteria that can grow in cells. Acquired immunity can be obtained by vaccinating pathogens or their toxins as immunogens, and this immunity is referred to as artificial immunity. Using this method, E. Jenner discovered the process of vaccination that constitutes the basis of immunology. These days, various artificial immunizations are being developed, and studies on the application thereof for prevention of, for example, cancer, Acquired Immune Deficiency Syndrome (AIDS) and hypersensitivity reactions.

Mushrooms have been recognized as health foods, because they are rich in flavoring components and have low contents of proteins and lipids, whereas they contain large amounts of special nutrients, such as polysaccharides, vitamins and minerals. In recent years, these mushrooms have been found to have pharmacological effects, including anticancer activity, immune-enhancing effects and antioxidant effects, and thus have been frequently used as materials for medical drugs and health functional foods.

It was previously found that an *Inonotus Obliquus* extract promotes the proliferation of splenic and peritoneal cells, shows excellent activities for interleukins and nitric oxides (NOs), indicators of immunity and anticancer activities, exhibits the effect of inhibiting the proliferation of cancer cells, and is effective in the prevention of adult diseases, such as diseases caused by immune deficiency, and cancer diseases (Korean Patent Laid-Open Publication No. 2004-0042119). Also, it was found that a composition containing *Ganoderma Luciderm* fruit-body powder has excellent effects of enhancing immunity and lowering cholesterol levels, and thus is effective in enhancing immunity (Korean Patent Laid-Open Publication No. 2003-0082619). In addition, *Phellinus Linteus* is classified as *Phellinus baumii* in view of mycology, and it was found that a *Phellinus baumii* extract has activity for macrophages *in vivo* and specifically increases the activity of B cells among splenic immune cells to enhance immune activity (Korean Patent Registration No. 0623270).

In addition, it was found that a complex polysaccharide obtained by culturing one selected from among *Lentinus edodes*, *Ganoderma Luciderm*, *Inonotus Obliquus*, *Agaricus blazei*, *Phellinus Linteus*, *Coriolus versicolor* and *Poria Cocos* which belong to the class *Basidiomycetes* enhances immune activity (Korean Patent Registration No. 0491362). However, because this polysaccharide contains a single species of mushroom, it neither provides nor increases the efficacy of a variety of mushrooms.

Meanwhile, in conventional technologies of enhancing immune activity using mushroom extracts, immune activity is merely enhanced through mechanisms of action, including increases in interleukin and NO activities and the proliferation of B cells in the spleen. However, study results that immune activity can be enhanced by proliferation of hematopoietic stem cells (undifferentiated hematopoietic progenitor cells) are rare.

Accordingly, the present inventors have made many efforts to develop a technology of activating immunity by proliferation of hematopoietic cells and, as a result, have found that a composition prepared by mixing an *Inonotus Obliquus* extract, a *Ganoderma Luciderm* extract and a *Phellinus Linteus* extract and then adding thereto plant extracts, including a red ginseng concentrate, a propolis concentrate, an *Artemisia capillaris* extract, a *Curcuma longa* extract, a *Glycyrrhiza uralensis* extract and a porcine placenta extract, enhances immune cell activity by promoting the proliferation of hematopoietic stem cells, thereby completing the present invention.

### DISCLOSURE OF INVENTION

It is an object of the present invention to provide a composition for promoting the proliferation of hematopoietic stem cells, which contains an *Inonotus Obliquus* extract, a *Ganoderma Luciderm* extract, a *Phellinus Linteus* extract, a red ginseng concentrate, a propolis concentrate, an *Artemisia capillaris* extract, a *Curcuma longa* extract, a *Glycyrrhiza uralensis* extract and a porcine placenta extract. Another object of the present invention is to provide a functional food for promoting the proliferation of hematopoietic stem cells, which contains an *Inonotus Obliquus* extract, a *Ganoderma Luciderm* extract, a *Phellinus Linteus* extract, a red ginseng concentrate, a propolis concentrate, an *Artemisia capillaris* extract, a *Curcuma longa* extract, a *Glycyrrhiza uralensis* extract, a porcine placenta extract and liquid fructose.

To achieve the above objects, the present invention provides a composition for promoting the proliferation of hematopoietic stem cells, which contains an *Inonotus Obliquus* extract, a *Ganoderma Luciderm* extract, a *Phellinus Linteus* extract, a red ginseng concentrate, a propolis concentrate, an *Artemisia capillaris* extract, a *Curcuma longa* extract, a *Glycyrrhiza uralensis* extract and a porcine placenta extract.

The present invention also provides a functional food for promoting the proliferation of hematopoietic stem cells, which contains an *Inonotus Obliquus* extract, a *Ganoderma Luciderm* extract, a *Phellinus Linteus* extract, a red ginseng concentrate, a propolis concentrate, an *Artemisia capillaris* extract, a *Curcuma longa* extract, a *Glycyrrhiza uralensis* extract, a porcine placenta extract and liquid fructose.

The present invention also provides the use of an *Inonotus Obliquus* extract, a *Ganoderma Luciderm* extract, a *Phellinus Linteus* extract, a red ginseng concentrate, a propolis concentrate, an *Artemisia capillaris* extract, a *Curcuma longa* extract, a *Glycyrrhiza uralensis* extract and a porcine placenta extract for promoting the proliferation of hematopoietic stem cells.

The present invention also provides the use of an *Inonotus Obliquus* extract, a *Ganoderma Luciderm* extract, a *Phellinus Linteus* extract, a red ginseng concentrate, a propolis concentrate, an *Artemisia capillaris* extract, a *Curcuma longa* extract, a *Glycyrrhiza uralensis* extract and a porcine placenta extract for enhancing immune activity.

Other features and embodiments of the present invention will be more apparent from the following detailed descriptions and the appended claims.

### BEST MODE FOR CARRYING OUT THE INVENTION

In one aspect, the present invention is directed to a composition for promoting the proliferation of hematopoietic stem cells, which contains an *Inonotus Obliquus* extract, a *Ganoderma Luciderm* extract, a *Phellinus Linteus* extract, a red ginseng concentrate, a propolis concentrate, an *Artemisia capillaries* extract, a *Curcuma longa* extract, a *Glycyrrhiza uralensis* extract and a porcine placenta extract.

The present invention is based on the finding that, when a mixture of an *Inonotus Obliquus* extract, a *Ganoderma Luciderm* extract and a *Phellinus Lintels* extract is mixed with plant extracts that promotes the immune activity-enhancing effects of the mushroom extracts, the resulting mixed extract can enhance immune activity by promoting the proliferation of hematopoietic stem cells.

*Inonotus Obliquus* that is used in the present invention is a mushroom that grows on the white birch in the high-latitude Siberian province. It was known that there are little or no gastrointestinal cancer patients and diabetic patients among the natives who drink the boiled water of *Inonotus Obliquus,* like tea and coffee. Also, an extract *of Inonotus Obliquus* contain active ingredients, including polysaccharides, flavonoids, triterpenoid, inositol, acids, and alkaloids, and shows high gastrointestinal antiulcer activity, antitumor activity, blood gluclose lowering activity and SOD activity.

*Ganoderma Luciderm* that is used in the present invention is a mushroom that grows on the root of broad-leaved trees in the summer season. According to the Shen Nong Ben Cao Jing (earliest Chinese herbal materia medica), *Ganoderma Luciderm* is known to be effective for urination, liver protection, tonic promotion, arthritis treatment, and bronchitis treatment and has blood pressure lowering, hyperlipidemia relief, blood glucose lowering and antitumor effects.

*Phellinus Linteus* that is used in the present invention is a mushroom belonging to the class *Basidiomycetes*, the order *Aphyllophorales* and the family *Phellinaceae*. It enhances immune function after surgery of stomach cancer, esophageal cancer, duodenal cancer, colon cancer, rectal cancer or liver cancer. According to the Bon-Cho-Kang-Mok (Chinese medicinal plant book), *Phellinus Lintels* is known to be effective for uterine bleeding, leucorrhea, and intestinal bleeding, activate the functions of the five viscera, and show the effect of counteracting poison.

Red ginseng that is used in the present invention is a ginseng product obtained by steaming non-dried ginseng and drying the steamed ginseng. Like white ginseng, red ginseng contains glucoside, ginseng flavor components, polyacetylene-based compounds, nitrogen-containing components, vitamin B, microelements, enzymes, antioxidant substances, organic acids and amino acids. It has sedative and stimulant effects on the central nerve system and acts on the circulatory system to prevent hypertension or arteriolosclerosis. Also, it has hematopoietic action, lowers blood glucose levels, protects the liver, and acts on the endocrine system to promote reproductive function indirectly. In addition, it has anti-inflammatory and antitumor, provides protection against radioactive rays, and has the effects of protecting skin and making the skin soft.

Propolis that is used in the present invention is also called Russian penicillin or natural penicillin. It has anti-inflammatory, antioxidant and immune-enhancing effects, is most rich in organic substances and minerals (inorganic salts), and contains minerals, vitamins, amino acids, fats, organic acids, flavonoids and the like, which play an important role in cellular metabolism, as well as terpenes having anticancer activity.

*Artemisia capillaries* that is used in the present invention contains dimethylesculetin, scopoletin, essential oil, aromatic oxycarbonic acids, flavonoids, oxycoumarin and the like. It has excellent effects on choleretic action, liver detoxification, excretion of toxic substances, improvement of liver function, treatment of liver diseases, and prevention of cardiovascular diseases, including hypertension, obesity and stroke.

*Curcuma longa* that is used in the present invention refers to one obtained by steaming and drying the tuberous root of *Curcuma longa* of the family *Zingiberaceae* with or without removal of the periderm. It circulates "Ki" and assists in blood circulation to treat menstrual pain, menstrual irregularity and flank pain. Also, it is effective in treating hematemesis, nasal bleeding and bloody urine, clarifying mentality, promoting bile excretion, and treating gallbladder stones. Also, it contains angiogenesis-inhibiting substances and thus has excellent effects on the inhibition of carcinogenesis and the promotion of bile excretion. In addition, *Curcuma longa* contains the yellow pigment curcumin that is a potent antioxidant substance (removal of reactive oxygen species) and swerves to prevent the oxidation of cells and reduce inflammation, preventing dementia or delaying the progress of dementia.

*Glycyrrhiza uralensis* that is used in the present invention is a medicinal herb. According to the Dong-Eui-Bo-Gam (Korean traditional medical handbook), *Glycyrrhiza uralensis* is known to be effective in lowering fever in the five viscera and the six entrails, normalizing the physiology of eye, noise, mouth and ear, normalizing urination and defecation, circulating all blood vessels, strengthening muscles and bones, improving nutrient conditions, and neutralizing the poison of drugs.

Hematopoiesis is a series of developmental processes in which numerous mature blood cells having a very special function and a limited lifetime are produced and continuously supplied. The hematopoietic system is a very complex system consisting of hematopoietic precursor cells that can be aligned according to their developmental capacities. Mature blood cells of all lineages are derived from marrow tissue which includes cells at several stages of maturation. Blood cells produced in the marrow are destroyed in peripheral tissues after a certain time, and this process is continuously repeated throughout the life of animals. Hematopoiesis is maintained by hematopoietic stem cells (HSCs) that can proliferate and differentiate into cells of various lineages. Early hematopoietic stem cells are called "pluripotent hematopoietic stem cells" (PHSCs) that make hemopoietic precursor cells which can differentiate into various mature blood cells through several processes which have not yet been found. The hemopoietic precursor cells produce further differentiated cells at the next stage.

HSCs have two important characteristics: a self-renewal ability to produce differentiated progeny, and an ability to differentiate into all lineages of lymphohematopoietic cells. Studies on hematopoietic stem cells were performed since it was first found that bone marrow deficiency caused by irradiating a sublethal dose of radiation into animals could be treated by injecting normal bone marrow cells into the animals. From this first quantitative experiment on bone marrow recovery in mice systemically irradiated with radiation, the concept of hematopoietic precursor cells having the capability to form cell colonies of bone marrow cell ad red blood cell lineages in the spleen and bone marrow of animals irradiated with radiation was introduced. To understand the reconstitution of blood cells by pluripotent hematopoietic stem cells (PHSCs) having self-renewal ability and the developmental biological aspects of hematopoietic lineages, the separation of pluripotent hematopoietic stem cells is required. However, because these cells are present at a very low ratio, these cells were significantly difficult to separate. However, the introduction of flow cytometry and the development of monoclonal antibodies for hematopoietic stem cell marker antigens and differentiation antigens made it possible to separate HSCs and understand the properties and characteristics of the HSCs.

In the present invention, the composition for promoting the proliferation of hematopoietic stem cells may contain, based on 100 parts by weight of the *Inonotus Obliquus* extract, 5-50 parts by weight of the *Ganoderma Luciderm* extract, 0.5-30 parts by weight of the *Phellinus Linteus* extract, 0.5-30 parts by weight of the red ginseng concentrate, 0.5-30 parts by weight of the *Artemisia capillaries* extract, 0.5-50 parts by weight of the *Curcuma longa* extract, 0.5-50 parts by weight of the *Glycyrrhiza uralensis* extract and 0.5-30 parts by weight of the porcine placenta extract.

In the present invention, when the *Inonotus Obliquus* extract (solid content: 2%) was administered to gastric polyp patients and gastric ulcer patients in an amount of 8 ml per one time and three times every day, the gastric polyp was significantly relieved, and pain, vomiting and belching phenomena in the gastric ulcer patients disappeared. *Inonotus Obliquus* has SOD activity (ability to remove reactive oxygen species) that is at least three times higher than other mushrooms, and thus when *Inonotus Obliquus* is taken, it will eliminate reactive oxygen species *in vivo* to assist in enhancing the activities of all cells in the body. Various physiologically active substances have been isolated and purified from mushrooms, and among them, beta-glucan, glycoprotein, dietary fiber, antioxidant substances and the like were found to be major substances showing physiological activity. *Inonotus Obliquus* has a beta-glucan content (having an immune-enhancing effect) of 57 µg/10 g which is more than 10 times higher than that of *Phellinus Linteus*. Also, *Inonotus Obliquus* has SOD activity which is more than three times higher than other mushrooms. For this reason, in order to promote the proliferation or activity of hematopoietic stem cells which are progenitor immune cells, *Inonotus Obliquus* forms the highest portion of the composition of the present invention.

In the present invention, the *Ganoderma Luciderm* extract may be contained in the composition in an amount of 5-50 parts by weight, and preferably 5-15 parts by weight, based on 100 parts by weight of the *Inonotus Obliquus* extract. If the content of the *Ganoderma Luciderm* extract is out of the above range, the proliferation of hematopoietic stem cells will be insignificant.

In the present invention, it was found that the protein-bound polysaccharide of the *Ganoderma Luciderm* extract is involved in the differentiation and proliferation of hematopoietic stem cells, significantly increases the antibody producing ability of B cells and enhances the immune response of T cells. It was found that, when protein-bound polysaccharide extracted from the *Ganoderma Luciderm* mycelium was administered intraperitoneally, it had the effect of inhibiting metastatic lung cancer in mice. This effect was believed to be because the protein-bound polysaccharide promoted the secretion of interleukins by activation of macrophages or NK cells.

In the present invention, the *Phellinus Linteus* extract may be contained in the composition in an amount of 0.5-30 parts by weight, and preferably 0.5-10 parts by weight, based on 100 parts by weight of the *Inonotus Obliquus* extract. If the content of the *Phellinus Linteus* extract is out of the above range, the activity of T cells will be weakened so that the activity and immune activity of hematopoietic stem cells.

In the present invention, it was reported that the *Phellinus Linteus* extract enhances immunity through the activation of NK cells and increases the number of helper T cells by activating bone marrow-derived dendritic cells, thereby exhibiting an anticancer effect. It was found that a hot water extract of *Phellinus Linteus* significantly increases the activity of antibody-producing B cells and also increases the activity of macrophages to induce NO production, thereby exhibiting an anticancer effect.

In the present invention, the red ginseng concentrate may be contained in the composition in an amount of 0.5-30 parts by weight, and preferably 1-15 parts by weight, based on 100 parts by weight of the *Inonotus Obliquus* extract. If the content of the red ginseng concentrate is out of the above range, the activity of hematopoietic stem cell will decrease and the activity of T-cells will decrease, so that immune activity will be weakened.

In the present invention, the propolis concentrate may be contained in the composition in an amount of 0.5-30 parts by weight, and preferably 0.5-10 parts by weight, based on 100 parts by weight of the *Inonotus Obliquus* extract. If the content of the propolis concentrate is out of the above range, the antioxidant and antibacterial activity of the composition will be reduced.

In the present invention, the *Artemisia capillaries* extract may be contained in the composition in an amount of 0.5-30 parts by weight, and preferably 1-20 parts by weight, based on 100 parts by weight of the *Inonotus Obliquus* extract. If the content of the *Artemisia capillaries* extract is out of the above range, the cell proliferation activity of the composition will be reduced and the liver detoxification activity of the composition will be reduced.

In the present invention, the *Curcuma longa* extract may be contained in the composition in an amount of 0.5-50 parts by weight, and preferably 1-30 parts by weight, based on 100 parts by weight of the *Inonotus Obliquus* extract. If the content of the *Curcuma longa* extract is out of the above range, the antioxidant activity of the composition will be reduced so that the activity of hematopoietic stem cells will be reduced.

In the present invention, the *Glycyrrhiza uralensis* extract may be contained in the composition in an amount of 0.5-50 parts by weight, and preferably 0.5-20 parts by weight, based on 100 parts by weight of the *Inonotus Obliquus* extract. If the content of the *Glycyrrhiza uralensis* extract is out of the above range, the anti-inflammatory activity and liver detoxification activity of the composition will be reduced.

In the present invention, the porcine placenta extract may be contained in an amount of 0.5-30 parts by weight, and preferably 1-20 parts by weight, based on 100 parts by weight of the *Inonotus Obliquus* extract. If the content of the porcine placenta extract is out of the above range, the cell growth promoting activity of the composition will be reduced.

In the present invention, red ginseng has effects on the enhancement of immune activity, recovery from fatigue, and the improvement of blood circulation, and *Artemisia capillaries* promotes cell growth and improve liver function. Also, *Curcuma longa* has an antioxidant effect and is effective in inhibiting carcinogenesis. Also, *Glycyrrhiza uralensis* has liver detoxification and antibacterial effects, and porcine placenta promotes cell growth and has the effects of enhancing immune activity and inhibiting aging. Thus, these substances are added to the mushroom extracts to provide a composition for promoting the proliferation of hematopoietic stem cells, which has excellent effects on the enhancement of immune activity and the proliferation of hematopoietic stem cells.

According to the present invention, the composition for promoting the proliferation of hematopoietic stem cells can be prepared through a method comprising the steps of: (i) extracting *Inonotus Obliquus* in purified water at 50~60 °C for 4-8 hours, extracting each of *Ganoderma Luciderm* and *Phellinus Linteus* in purified water at 70~80 °C for 8-16 hours, filtering each of the extracts through a 1-10 µm filter, concentrating each of the filtrates under reduced pressure at 60~80 °C, and mixing the filtered extracts with each other; (ii) extracting each of *Artemisia capillaries*, *Curcuma longa* and *Glycyrrhiza uralensis* in purified water at 70~80 °C for 8-16 hours, filtering each of the extracts through a 1-10 µm, concentrating each of the filtrates under reduced pressure at 60~80 °C; (iii) adding the extracts of step (ii) to the mixed extract of step (i); and (iv) adding to the extract mixture of step (iii) a red ginseng concentrate, a propolis concentrate and a porcine placenta extract.

The inventive composition for promoting the proliferation of hematopoietic stem cells may be provided in the form of a pharmaceutical composition or a food composition.

The inventive pharmaceutical composition, comprising an *Inonotus Obliquus* extract, a *Ganoderma Luciderm* extract, a *Phellinus Linteus* extract, a red ginseng concentrate, a propolis concentrate, an *Artemisia capillaris* extract, a *Curcuma longa* extract, a *Glycyrrhiza uralensis* extract and a porcine placenta extract, may additionally comprise conventional carriers, excipients or diluents. Examples of suitable carriers, excipients and diluents include lactose, dextrose, sucrose, sorbitol, mannitol, xylitol, erythritol, maltitol, starches, acacia rubber, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate and mineral oil. The formulations may additionally include fillers, anti-aglutinating agents, lubricating agents, wetting agents, flavoring agents, emulsifiers, preservatives and the like. The compositions of the present invention may be formulated so as to provide quick, sustained or delayed release of the active ingredient after their administration to a patient by employing any of the procedures well known in the art. The formulations may be in the form of tablets, powders, pills, sachets, elixirs, suspensions, emulsions, solutions, syrups, aerosols, soft or hard gelatin capsules, sterile injectable solutions and sterilized powders. The composition of the present invention may be administered via various routes, including oral, transdermal, subcutaneous, intravenous or intramuscular routes. The dose of the composition, comprising the extracts, which is to be administered into the human body, may vary depending on the patient's age, weight and sex, the mode of administration, physical conditions, and the severity of disease. The dose is generally 80-240 ml/day, and preferably 80-160 ml/day, for a 70 kg adult. Also, the composition of the present invention may be administered once or three times at certain time intervals depending on the determination of physicians or pharmacists. In addition, the dose of the composition may vary depending on the patient's age, sex, weight and physical conditions.

The food composition of the present invention can be prepared in the form of various kinds of foods, for example, in the form of a beverage composition. Also, the food composition may contain liquid fructose.

In another aspect, the present invention is directed to a functional food for promoting the proliferation of hematopoietic stem cells, which contains an *Inonotus Obliquus* extract, a *Ganoderma Luciderm* extract, a *Phellinus Linteus* extract, a red ginseng concentrate, a propolis concentrate, an *Artemisia capillaris* extract, a *Curcuma longa* extract, a *Glycyrrhiza uralensis* extract, a porcine placenta extract and liquid fructose.

As used herein, the term "functional food" is the same term as food for special health use (FoSHU) and refers to a food having high medicinal and medical effects, which is processed to effectively exert a body-regulating function and to supply nutrients. As used herein, the term "health food" refers to a food that positively maintains or improves health compared to general food, and the term "health supplement food" refers to a food to be used as a health supplement. In some cases, the terms "functional food", "health food" and "health supplement food" are used interchangeably with each other. The food can be prepared in various forms so as to provide a useful effect of promoting the proliferation of hematopoietic stem cells.

Thus, the present invention relates to a functional food for promoting the proliferation of hematopoietic stem cells, which contains, based on 100 parts by weight of the *Inonotus Obliquus* extract, 5-50 parts by weight of the *Ganoderma Luciderm* extract, 0.5-30 parts by weight of the *Phellinus Linteus* extract, 0.5-30 parts by weight of the red ginseng concentrate, 0.5-30 parts by weight of the *Artemisia capillaries* extract, 0.5-50 parts by weight of the *Curcuma longa* extract, 0.5-50 parts by weight of the *Glycyrrhiza uralensis* extract, 0.5-30 parts by weight of the porcine placenta extract and 20-80 parts by weight of liquid fructose.

In the present invention, the liquid fructose may be contained in the functional food in an amount of 20-80 parts by weight, and preferably 30-60 parts by weight, based on 100 parts by weight of the *Inonotus Obliquus* extract. If the content of the liquid fructose is out of the above range, the preference for the taste of the functional food as a beverage product will be reduced.

### EXAMPLES

Hereinafter, the present invention will be described in further detail with reference to examples. It will be obvious to a person having ordinary skill in the art that these examples are illustrative purposes only and are not to be construed to limit the scope of the present invention.

### Example 1: Preparation of beverage composition for promoting proliferation of hematopoietic stem cells

*Inonotus Obliquus* (produced in Russia) was extracted in purified water (weight corresponding to 10 times the dry weight of the mushroom) at 50~60 °C for 4-8 hours, and each of *Ganoderma Luciderm* (produced in Yeoju, Korea) and *Phellinus Linteus* (DooWon Agricultural Co., Korea) was extracted in purified water (weight corresponding to 10 times the dry weight of the mushroom) 70~80 °C for 8-16 hours. Each of the extracts was filtered through a 1-10-µm filter, and concentrated under reduced pressure at 60~80 °C. Then, 15 g of the *Inonotus Obliquus* extract (solid content: 5%), 1.3 g of the *Ganoderma Luciderm* extract (solid content: 5%) and 0.7 g of the *Phellinus Linteus* extract (solid content: 5%) were mixed with each other in purified water. Meanwhile, each of *Artemisia capillaries*, *Curcuma longa* and *Glycyrrhiza uralensis* was extracted in purified water at 70~80 °C for 8-16 hours, and each of the extracts was filtered through a 1-10-µm filter and concentrated under reduced at 60-80 °C, and then 0.6 g of the *Artemisia capillaries* (solid content: 5%), 1.0 g of the *Curcuma longa* extract (solid content: 5%) and 1.0 g of the *Glycyrrhiza uralensis* extract (solid content: 32%) were added to the above-prepared extract mixture. To the resulting extract mixture, 0.6 g of a red ginseng concentrate (70 mg saponin/g), 0.3 g of a propolis concentrate (1% flavonoid), 0.6 g of a porcine placenta extract (solid content: 55%) and 7.5 g of liquid fructose (F-55) were added, thereby preparing a composition for promoting the proliferation of hematopoietic stem cells (Table 1).

**<Table 1> Amounts of components used in preparation of 100 g of beverage composition for promoting proliferation of hematopoietic stem cells**

| Component | *Inonotus Obliquus* extract | *Ganoderma Luciderm* extract | *Phellinus Linteus* extract | red ginseng concentrate | propolis concentrate | *Artemisia capillaries* extract |
|---|---|---|---|---|---|---|
| Amount | 15g | 1.3g | 0.7g | 0.6g | 0.3g | 0.6g |

| Components | *Curcuma longa* extract | *Glycyrrhiza uralensis* extract | porcine placenta extract | liquid fructose | purified water | |
|---|---|---|---|---|---|---|
| Amount | 1.0g | 1.0g | 0.6g | 7.5g | 71.4g | |

### Comparative Example 1: Preparation of placebo beverage

A placebo beverage was prepared by adding 30 g of an *Artemisia capillaries* extract (solid content: 1.5%) and 12.5 g of liquid fructose (F-55) to 57.5 g of purified water (Table 2).

**<Table 2> Amounts of components used in preparation of 100 g of placebo beverage**

| Component | *Artemisia capillaries* extract | liquid fructose | purified water |
|---|---|---|---|
| Amount | 30g | 12.5g | 57.5g |

### Test Example 1: Observation of immune-enhancing effect of composition for promoting the proliferation of hematopoietic stem cells

A test for the effect of promoting the proliferation of hematopoietic stem cells was carried out by causing test subjects to drink each of the beverage composition prepared in Example 1 and the placebo beverage prepared in Comparative Example 1.

The test subjects consisted of healthy volunteers (40-70 year old men and women). One week before the test, blood was sampled from the test subjects using the automatic serum analyzer TBA-20R (Toshiba, Japan), and GOT and GPT levels in the blood samples were analyzed. On the basis of the analysis results, severe liver disease patents having high GPT and GOT levels were excluded from the test. After the blood analysis, a total of 39 persons were selected and randomly divided into two groups: a group consisting of 27 persons who were to be administered with the beverage composition for promoting the proliferation of hematopoietic stem cells; and a group consisting of 12 persons who were to be administered with the placebo beverage. Then, the beverage composition for promoting the proliferation of hematopoietic stem cells and the placebo beverage was provided to the test subjects and administered for 4 weeks. The test subjects consisted of 33 women and 6 men. Each of the subjects was administered with 80 ml of each of the beverage composition and the placebo beverage twice (morning and evening) a day for a total of 4 weeks. During the 4 week test period, the subjects abstained from taking health foods and alcoholic drinks other than the beverage composition and the placebo beverage. The physical characteristics of the groups administered with each of the beverage composition and the placebo beverage are shown in Table 3 below.

**<Table 3> Physical characteristics of the group administered with the beverage composition for promoting proliferation of hematopoietic stem cells and the group administered with the placebo beverage**

| | | A group consisting of persons who were to be administered with the beverage composition for promoting the Proliferation of hematopoietic stem cells (N=27) | A group consisting of persons who were to be administered with the placebo beverage (N=12) | Total (N=39) |
|---|---|---|---|---|
| Sex | male | 23 | 10 | 33 |
| | female | 4 | 2 6 | |
| Age | 40 - 50 | 8 | 2 | 10 |
| | 50 - 60 | 15 | 6 | 21 |
| | > 60 | 4 | 4 | 8 |
| The frequency of taking alcoholic drinks | - | 21 | 10 | 31 |
| | ≤once/a week | 6 | 2 | 8 |
| | ≥once/a week | 0 | 0 | 0 |

Means and standard deviations were calculated on the data obtained, and the analysis of the data was carried out using SPSS 16.0 statistical program. To compare the difference between the administration periods of the beverage composition and the placebo beverage (before administration, 2 weeks after administration, and 4 weeks after administration) with respect to cell variables, paired t-test was performed. Also, to compare the difference between the groups administered with the beverage composition and the placebo beverage, independent t-test was performed, and p<0.05 was considered statistically significant.

### 1-1: Change in number of hematopoietic stem cells

As a peripheral blood leukocyte sample, 1×10⁶ cells/mℓ were prepared in a single cell suspension. In a control tube, 100 µℓ of the leukocyte sample and 10 µℓ of CD45-FITC were placed, and in a test tube, 100 µℓ of the leukocyte sample, 10 µℓ of CD45-FITC and 10 µℓ of CD34-PE 10 µℓ were placed, mixed with each other and allowed to react in a dark room at room temperature for 15 minutes. In each of the tubes, 2 mℓ of lysing solution (1X, BD, USA) was placed, stirred well and then allowed to react in a dark room at room temperature for 10 minutes. Each of the reaction samples was centrifuged (X 300g) at room temperature for 15 minutes, and the supernatant was removed. 2 mℓ of PBS was added to each of the tubes, stirred well and centrifuged (X 200g) for 5 minutes. The supernatant of each tube was removed, and the remaining material was stained with 0.5 mℓ of PBS (with 0.5% paraformaldehyde).

CD45 antigen-expressing cells, excluding CD45-nonexpressing cells and dead cells, were selected using the FACS analyzer FACScalibur and Cell Quest^{™} program (Becton Dickenson, USA) according to the ISHAGE method in the SSC (side scatter) vs CD45 FL-1 mode, and then CD34 antigen-expressing cells were selected using the SSC vs CD34 FL-2 mode. Among these cells, cells having low SSC and low or intermediate CD45 FL-1 and FSC were selected in the SSC vs CD45 F-L1 and FSC (forward scatter) vs SSC modes in order to prevent contamination of cells showing non-specific reactions. 100,000 CD45 ⁺ cells were analyzed, and the ratio (%) of CD34⁺ cells was calculated according to the following equation: (CD34⁺, low SSC, low to intermediate FSC and CD45 FL-1 cells/CD45⁺cells)×100. Also, the number of CD34⁺ cells was calculated according to the following equation: number of leukocytes ×CD34 (%).

To separate peripheral blood leukocytes, 10 ml of peripheral blood collected from the antebracheal vein of the test subjects was diluted in 10 ml of D-PBS. In 50 ml of a conical tube, 15 ml of Ficoll-Plaque^{™}plus (GE Healthcare) was placed, 20 ml of the diluted blood was added slowly thereto, and then centrifuged (X 700g) for 50 minutes, and the supernatant (buffy coat) was collected and washed twice with D-PBS, thus separating peripheral blood leukocytes. Using the separated cells, the change in the number of hematopoietic stem cells before and after administration of the beverage composition for promoting the proliferation of hematopoietic stem cells and the placebo beverage was observed.

As a result, the number of hematopoietic stem cells in the group administered with the beverage composition for promoting the proliferation of hematopoietic stem cells was significantly increased from an average of 3.89 cells per 100,000 leukocytes before administration to 8.78 cells (p<0.01) 2 weeks after administration and 7.14 cells (p<0.001) 4 weeks after administration, and this increase was statistically significant. However, the number of hematopoietic stem cells in the group administered with the placebo beverage was significantly decreased from an average of 8.67 cells per 100,000 leukocytes before administration to 2.75 cells (p<0.01) 2 weeks after administration and 4.38 cells (p<0.05) 4 weeks after administration. Meanwhile, using independent t-test, the change in hematopoietic stem cells between the group administered with the placebo beverage and the group administered with the beverage composition for promoting the proliferation of hematopoietic stem cells for each administration period was analyzed. As a result, a significant difference (p<0.05) between the two groups was already shown before administration, and thus the significant difference between the two groups 2 weeks and 4 weeks after administration could not be acknowledged. In the group administered with the beverage composition for promoting the proliferation of hematopoietic stem cells, the number of persons who showed an increase in the number of hematopoietic stem cells 4 weeks after administration was 15 of 27 persons, the number of persons who showed a decrease in the number of hematopoietic stem cells was 7, and the number of persons who showed little or no change in the number of hematopoietic stem cells was 5. Also, in the group administered with the beverage composition for promoting the proliferation of hematopoietic stem cells, side effects did not appear, except that one woman of 27 persons underwent light abdominal pain at the initial stage of administration.

**<Table 4> Number of hematopoietic stem cells after administration of beverage composition for promoting proliferation of hematopoietic stem cells**

| | A group consisting of persons who were to be administered with the placebo beverage (12) | A group consisting of persons who were to be administered with the beverage composition for promoting the Proliferation of hematopoietic stem cells (27) | p-valuel¹⁾ |
|---|---|---|---|
| | Cell No.(/10⁵ Leukocytes, Mean±SD) | | |
| before administration | 8.67±4.66 | 3.89±2.53 | 0.011* |
| 2 weeks after administration | 2.75±2.49 | 8.78±9.23 | 0.004** |
| 4 weeks after administration | 4.38±3.20 | 7.14±3.19 | 0.014* |
| p-value²⁾ 2 weeks after | 0.001** | 0.008** | |
| p-value²⁾ 4 weeks after | 0.021** | 0.000*** | |

| | | | |
|---|---|---|---|
| 1) Comparison between the beverage composition for promoting proliferation of hematopoietic stem cells and the placebo beverage by independent t-test 2) Comparison between before and after administration by paired t-test * p<0.05, ** p<0.01, *** p<0.001 | | | |

### 1-2: Change in number of lymphocytes

Using the automatic blood cell analyzer Beckman Coulter STKS (Beckman, USA), the number of lymphocytes in the blood collected from the subjects was measured. As a result, in the group administered with the beverage composition for promoting the proliferation of hematopoietic stem cells, the number of lymphocytes in the peripheral blood was decreased from an average of 1,850 cells/#µℓ to 1,780 cells/#µℓ at 2 weeks after administration and 1,720 cells/#µℓ at 4 weeks after administration, but this decrease was not statistically significant. On the other hand, in the group administered with the placebo beverage, the change in the number of lymphocytes did not appear even 2 weeks and 4 weeks after administration (Table 5).

**<Table 5> Number of lymphocytes after administration of beverage composition for promoting proliferation of hematopoietic stem cells**

| | A group consisting of persons who were to be administered with the placebo beverage (12) | A group consisting of persons who were to be administered with the beverage composition for promoting the Proliferation of hematopoietic stem cells (27) | p-value¹⁾ |
|---|---|---|---|
| | Cell No.(/10³/µℓ, Mean±SD) | | |
| before administration | 1.08±0.59 | 1.85±0.40 | 0.795 |
| 2 weeks after administration | 1.70±0.54 | 1.78±0.46 | 0.639 |
| 4 weeks after administration | 1.81±0.42 | 1.72±±0.64 | 0.646 |
| p-value²⁾ 2 weeks after | 0.454 | 0.278 | |
| p-value²⁾ 4 weeks after | 0.949 | 0.125 | |

| | | | |
|---|---|---|---|
| 1) Comparison between the beverage composition for promoting proliferation of hematopoietic stem cells and the placebo beverage by independent t-test 2) Comparison between before and after administration by paired t-test * p<0.05, ** p<0.01, *** p<0.001 | | | |

### 1-3: Changes in ratios of T cell subtypes (CD4⁺, CD8⁺, CD4⁺/CD8⁺)

As a peripheral leukocyte sample, 1 × 10⁶ cells/mℓ were prepared in a single cell suspension. To the leukocyte sample, 1% paraformaldehyde was added, and the cells were immobilized at 4 °C for 1 hour. Then, each of 2.5% normal goat serum (Vector, USA) and 2.5% normal horse serum (Vector, USA) was added thereto and allowed to react at 4 °C for 1 hour. 40 µℓ of the reacted sample was dispensed into each well of a 96-well plate. Then, 4 uℓ of CD3 antibody (BD, USA) was added to each well. 4 uℓ of each of CD4 antibody (BD, USA) and CD8 antibody (BD, USA) was added to CD4-labelded well and CD8-labeled well, and each of the samples was double-stained at 4 °C for 1 hour. Then, each of the samples was washed three times with DPBS. Then, each sample was stained in a FACS tube.

Using the FACS analyzer FACScalibur and Cell Quest^{™} program (Becton Dickenson, USA), quartering was performed based on the negative control density plot of CD4 FL-1 (y-axis) and CD8 FL-4 (y-axis) versus CD3 FL-2 (x-axis). Analysis for each of CD3/CD4 and CD3/CD8 was performed, and the value appearing in 2/4 quarter was taken as the ratio of helper T cells (CD3⁺/CD4⁺). In the same manner, the value appearing in 2/4 quarter was taken as the ratio of cytotoxic T cells (CD3⁺/CD8⁺). Based on the analysis results for the ratio of each of CD4⁺cells and CD8⁺ cells, the CD4:CD8 ratio was analyzed.

### 1) CD4⁺ T cells (helper T cells)

In the group administered with the beverage composition for promoting the proliferation of hematopoietic stem cells, the ratio of CD4⁺ T cells was insignificantly changed from 42.35% before administration to 42.41% at 4 week after administration. Also, in the group administered with the placebo beverage, there was no significant difference in the ratio of CD4⁺ T cells between before and after administration. The change in ratio of CD4⁺ T cells for each administration period did not differ between the group administered with the placebo beverage and the group administered with the beverage composition for promoting the proliferation of hematopoietic stem cells (Table 6).

### 2) CD8⁺ T cells (cytotoxic T cells)

In the group administered with the beverage composition for promoting the proliferation of hematopoietic stem cells, the ratio of CD8⁺ T cells was significantly increased from 22.25% before administration to 24.08% at 4 weeks after administration (p<0.05), and in the group administered with the placebo beverage, the ratio of CD8⁺ T cells was also significantly increased from 19.88% before administration to 23.14% at 4 weeks after administration (p<0.01). It could not be considered that the ratio of CD8⁺ T cells was statistically significantly changed due to administration of the beverage composition for promoting the proliferation of hematopoietic stem cells (Table 6). The change in number of CD8⁺ T cells during each administration period did not differ between the group administered with the placebo beverage and the group administered with the beverage composition for promoting the proliferation of hematopoietic stem cells (Table 6).

### 3) Change in ratio of CD4⁺/CD8⁺ ratio (%)

In the group administered with the beverage composition for promoting the proliferation of hematopoietic stem cells, the CD4⁺/CD8⁺ ratio was decreased from 2.21% before administration to 2.16% at 2 weeks after administration and 2.06% at 4 weeks after administration, but this difference was not statistically significant. In the group administered with the placebo beverage, the CD4⁺/CD8⁺ ratio was decreased from 2.53% before administration to 2.32% at 2 weeks after administration and 2.19% at 4 weeks after administration, but this difference was not statistically significant. The CD4⁺/CD8⁺ T cell ratio is used as an important indicator of cell-mediated immunity. The CD4⁺/CD8⁺ T cell ratio is about 1.8-2.2 for normal persons, and when it is decreased to 1.5 or less, immunity can be weakened so that viral infection can be increased. In the group administered with the beverage composition for promoting the proliferation of hematopoietic stem cells, the CD4⁺/CD8⁺ T cell ratio before and after administration was maintained at 2.0 or more that is within the range of values for normal persons (Table 6). Such results indicated that 4-week administration of the beverage composition for promoting the proliferation of hematopoietic stem cells had a positive effect on the immune function of T cells.

**<Table 6> Ratios of T cell subtypes after administration of beverage composition for promoting proliferation of hematopoietic stem cells**

| | A group consisting of persons who were to be administered with the placebo beverage (12) | A group consisting of persons who were to be administered with the beverage composition for promoting the Proliferation of hematopoietic stem cells (27) | p-valuel¹⁾ |
|---|---|---|---|
| | Cell Ratio.(%, Mean±SD) | | |
| CD4+ (%) | | | |
| before administration | 39.63±12.32 | 42.35±7.38 | 0.434 |
| 2 weeks after administration | 39.98±7.06 | 41.94±8.70 | 0.498 |
| 4 weeks after administration | 40.55±8.44 | 42.41±7.63 | 0.486 |
| p-value²⁾ 2 weeks after | 0.885 | 0.619 | |
| p-value²⁾ 4 weeks after | 0.636 | 0.607 | |

| CD8+ (%) | | | |
|---|---|---|---|
| before administration | 19.88±8.27 | 22.25±7.51 | 0.437 |
| 2 weeks after administration | 22.00±9.05 | 23.08±8.24 | 0.717 |
| 4 weeks after administration | 23.14±8.88 | 24.08±7.52 | 0.727 |
| p-value²⁾ 2 weeks after | 0.014* | 0.050 | |
| p-value²⁾ 4 weeks after | 0.002** | 0.012* | |

| CD4+/CD8+ (%) | | | |
|---|---|---|---|
| before administration | 2.53±2.13 | 2.21±1.16 | 0.579 |
| 2 weeks after administration | 2.32±1.65 | 2.16±1.27 | 0.747 |
| 4 weeks after administration | 2.19±1.54 | 2.06±1.30 | 0.778 |
| p-value²⁾ 2 weeks after | 0.268 | 0.381 | |
| p-value²⁾ 4 weeks after | 0.206 | 0.281 | |

| | | | |
|---|---|---|---|
| 1) Comparison between the beverage composition for promoting proliferation of hematopoietic stem cells and the placebo beverage by independent t-test 2) Comparison between before and after administration by paired t-test * p<0.05, ** p<0.01, *** p<0.001 | | | |

### 1-4: Change in ratio (%) of NK cells

As a peripheral blood leukocyte sample, 1×10⁶ cells/mℓ were prepared in a single cell suspension. 1% paraformaldehyde was added to the leukocyte sample at a concentration of 1%, and the cells were immobilized at 4 °C for 1 hour. Then, each of 2.5% normal goat serum (Vector, USA) and 2.5% normal horse serum (Vector, USA) was added thereto and allowed to react at 4 °C for 1 hour. 40 µℓ of the reacted sample was dispensed into each well of a 96-well plate. Then, 4 µℓ of CD3 antibody (BD, USA) was added to each well. 4 µℓ of CD56 antibody (BD, USA) was added to each CD56-labeled well, and the sample was double-stained at 4 °C for 1 hour. Then, the sample was washed three times with DPBS. Then, the reaction sample was stained in a FACS tube.

Using the FACS analyzer FACScalibur and Cell Quest^{™} program (Becton Dickenson, USA), quartering was performed based on the negative control density plot of CD56 FL-4 (y-axis) versus CD3 FL-2 (x-axis) in order to analyze the ratio of NK cells. The sample which has been immunofluorescence-stained with CD3/CD56 was analyzed, and the value appearing in 1/4 quarter was taken as the ratio of natural killer cells (CD3⁻/CD56⁺).

In the group administered with the beverage composition for promoting the proliferation of hematopoietic stem cells, the ratio of NK cells (CD3⁻, CD56⁺) in the peripheral blood lymphocytes was decreased from 17.54% before administration of the composition to 17.01% at 4 weeks after administration, but this decrease was not statistically significant. Also, in the group administered with the placebo beverage, the ratio of NK cells (CD3⁻, CD56⁺) in the peripheral blood lymphocytes was decreased at 4 weeks after administration compared to before administration, but this decrease was not statistically significant (Table 7).

**<Table 7> Ratio of NK cells after administration of beverage composition for promoting proliferation of hematopoietic stem cells**

| | A group consisting of persons who were to be administered with the placebo beverage (12) | A group consisting of persons who were to be administered with the beverage composition for promoting the Proliferation of hematopoietic stem cells (27) | p-valuel¹⁾ |
|---|---|---|---|
| | Cell Ratio. (%, Mean±SD) | | |
| before administration | 19.99±10.24 | 17.54±8.72 | 0.523 |
| 2 weeks after administration | 17.77±6.83 | 17.53±9.51 | 0.938 |
| 4 weeks after administration | 18.65±8.54 | 17.01±7.71 | 0.546 |
| p-value²⁾ 2 weeks after | 0.174 | 0.987 | |
| p-value²⁾ 4 weeks after | 0.554 | 0.443 | |

| | | | |
|---|---|---|---|
| 1) Comparison between the beverage composition for promoting proliferation of hematopoietic stem cells and the placebo beverage by independent t-test 2) Comparison between before and after administration by paired t-test * p<0.05, ** p<0.01, *** p<0.001 | | | |

### Comparative Example 2: Comparison of immune-enhancing mechanism

The immune-enhancing mechanism of the beverage composition of Example 1 for promoting the proliferation of hematopoietic stem cells, which contains the *Inonotus Obliquus* extract, the *Ganoderma Luciderm* extract and the *Phellinus Linteus* extract, was compared with those of prior-art mushroom extracts. It was reported that the *Inonotus Obliquus* extract disclosed in Korean Patent Laid-Open Publication No. 2004-0042119 promotes the proliferation of splenic and peritoneal cells, shows excellent activities for interleukin and NO and has an excellent effect on the inhibition of proliferation of cancer cells. Also, it was reported that *Ganoderma Luciderm* disclosed in Korean Patent Laid-Open Publication No. 2003-0082619 has excellent effects of enhancing immunity and lowering cholesterol levels, and the *Phellinus Linteus* extract disclosed in Korean Patent Registration No. 623270 specifically increases the activity of B cells among immune cells in the spleen to enhance immune activity. However, the enhancement of immune activity by proliferation of hematopoietic stem cells has not yet been reported.

However, when the changes occurring before and after administration of the inventive beverage composition containing the *Inonotus Obliquus* extract, the *Ganoderma Luciderm* extract and the *Phellinus Linteus* extract, prepared in Example 1, were observed, the number of hematopoietic stem cells in peripheral blood was significantly increased after administration of the composition, suggesting that the composition of the present invention assists in enhancing general immune activity by normalizing whole blood cells. Thus, it could be seen that the composition of the present invention has excellent effects compared to the single mushroom extracts according to the prior art (Table 8).

**<Table 8> Comparison of immunity enhancing mechanism between mushroom extracts**

| Number | mushroom extracts | use | mechanism |
|---|---|---|---|
| Korean Patent Laid-Open Publication No. 2004-0042119 | *Inonotus Obliquus* | Activation of immunity | proliferation of splenic cells, activiation of interleukin, enhancement of NO production capacity |
| Korean Patent Laid-Open Publication No. 2003-0082619 | *Ganoderma Luciderm* | Activation of immunity | activiation of T-cells, activiation of B-cells and activation of macrophages |
| Korean Patent Registration No. 623270 | *Phellinus Linteus* | Activation of immunity | activiation of B-cells |
| Example 1 | *Inonotus Obliquus, Ganoderma Luciderm* and *Phellinus Linteus* | Activation of immunity | Increasing Number of hematopoietic stem cells |

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

### INDUSTRIAL APPLICABILITY

The composition and functional food of the invention assists in enhancing immune activity by significantly promoting the proliferation of hematopoietic stem cells, and shows positive effects on the increase in anticancer activity, anti-inflammatory activity and cell activity, the regeneration of damaged tissue and the prevention of cardiovascular diseases, through the proliferation of hematopoietic stem cells and the enhancement of immune activity.

## Claims

1. A composition for promoting the proliferation of hematopoietic stem cells, which contains an *Inonotus Obliquus* extract, a *Ganoderma Luciderm* extract, a *Phellinus Linteus* extract, a red ginseng concentrate, a propolis concentrate, an *Artemisia capillaris* extract, a *Curcuma longa* extract, a *Glycyrrhiza uralensis* extract and a porcine placenta extract.

2. The composition for promoting the proliferation of hematopoietic stem cells of claim 1, wherein the composition contains 5-50 parts by weight of the *Ganoderma Luciderm* extract, 0.5-30 parts by weight of the *Phellinus Linteus* extract, 0.5-30 parts by weight of the red ginseng concentrate, 0.5-30 parts by weight of the *Artemisia capillaries* extract, 0.5-50 parts by weight of the *Curcuma longa* extract, 0.5-50 parts by weight of the *Glycyrrhiza uralensis* extract and 0.5-30 parts by weight of the porcine placenta extract based on 100 parts by weight of the *Inonotus Obliquus* extract.

3. A functional food for promoting the proliferation of hematopoietic stem cells, which contains an *Inonotus Obliquus* extract, a *Ganoderma Luciderm* extract, a *Phellinus Linteus* extract, a red ginseng concentrate, a propolis concentrate, an *Artemisia capillaris* extract, a *Curcuma longa* extract, a *Glycyrrhiza uralensis* extract, a porcine placenta extract and liquid fructose.

4. The functional food for promoting the proliferation of hematopoietic stem cells of claim 3, wherein the composition contains 5-50 parts by weight of the *Ganoderma Luciderm* extract, 0.5-30 parts by weight of the *Phellinus Linteus* extract, 0.5-30 parts by weight of the red ginseng concentrate, 0.5-30 parts by weight of the *Artemisia capillaries* extract, 0.5-50 parts by weight of the *Curcuma longa* extract, 0.5-50 parts by weight of the *Glycyrrhiza uralensis* extract and 0.5-30 parts by weight of the porcine placenta extract based on 100 parts by weight of the *Inonotus Obliquus* extract.
